# EUROPEAN PATENT APPLICATION

(11) **EP 4 648 557 A1**
(43) Date of publication of application: **12.11.2025**
(21) Application number: 24174700.5
(22) Date of filing: 08.05.2024
(51) Int. Cl.: H05K 5/02, A61B 5/00, H01R 13/518

(54) **CONNECTOR INTERFACE AND PATIENT MONITORING MODULE**

(71) Applicant: Koninklijke Philips N.V., 5656 AG Eindhoven (NL)
(72) Inventor: VOGELMANN, Maximilian, Eindhoven (NL); RITTER, Jasmin, Eindhoven (NL); SCHMITT-RADLOFF, Ulrich, 5656AG Eindhoven (NL); HOETZEL, Julia Maria, Eindhoven (NL)
(74) Representative: Philips Intellectual Property & Standards

(57) **Abstract**

The present disclosure relates to a connector interface (12) for a patient monitoring module (10) and to a patient monitoring module (10) that is equipped with such a connector interface (12). The connector interface (12) comprises a case part (14) having an outer side (50) and an inner side (52), at least one connector opening (56, 58, 60) formed in the case part (14) and extending between the outer side (50) and the inner side (52), at least one connector unit (20, 22, 24, 26, 28; 420, 422, 424, 426, 428) that is arranged at the at least one connector opening (56, 58, 60), and a mounting piece (70, 470) having at least one seat (74, 76) for the at least one connector unit (20, 22, 24, 26, 28; 420, 422, 424, 426, 428). The connector unit (20, 22, 24, 26, 28; 420, 422, 424, 426, 428) is accessible through the connector opening (56, 58, 60) from the outer side (50) in an engagement direction (36). The mounting piece (70, 470) is attached to the case part (14) at the inner side (52) thereof, thereby securing the connector unit (20, 22, 24, 26, 28; 420, 422, 424, 426, 428) in a mounting position with respect to the case part (14).

## Description

### FIELD OF THE INVENTION

The present disclosure relates to a connector interface for a patient monitoring module, and to a patient monitoring module that is equipped with such an interface. More generally, the present disclosure relates to medical devices, in particular to patient monitoring devices, at least in certain embodiments.

### BACKGROUND OF THE INVENTION

Patient monitors are widely used in medical environments, e.g., in ICUs (intensive care units) or generally in hospitals, to monitor and visualize patient-related data, in particular clinical data such as vital signs, scores and other information related to the patient's health condition. There are different types of patient monitors available including portable devices, accessory devices, stationary stand-alone devices (such as bedside monitors), and other implementations that are operable on conventional computers (including server-client environments).

Application fields for patient monitors and respective patient monitor modules include intensive care, bedside care and/or mobile care. Patient monitoring modules that can serve as measurement interfaces inside patient monitors but can also serve as external/stand-alone device (e.g., so-called measurement extensions). For instance, a mobile patient monitor can be directly docked to a stationary patient monitor to synchronize them with respect to medical data and/or to charge the battery of the mobile patient monitor.

WO 2016/188741 A1 generally relates to patient monitors. WO 2015/094248 A1 discloses a modular rack that enables a patient monitor to connect to different vital sign monitor connectors. US 2020/0352525 A1 relates to a multi-parameter patient monitoring device rack that can dock a plurality of patient monitor modules and that is configured to communicate with a separate display unit.

Patient monitors have to be fed with relevant information, involving measurement parameters that represent vital signs information and similar health information. To this end, patient monitor modules are typically equipped with connector interfaces including one or more patient connector units (e.g., sockets) that can be used to connect sensors and consumables, probes, lines, extension cables, leads etc. that deliver patient health information, such as blood pressure, respiration rate, pulse rate, ECG data, temperature, CO₂ measurements, functional arterial oxygen saturation (SpOz) measurements, etc.

Patient monitors often come with a modular configuration that makes it possible to adapt the patient monitor device specifically to the present monitoring task. To this end, patient monitoring modules may be equipped with connector interfaces that make it possible to connect one or more probes, sensors, and the like to the patient monitoring module at the same time. It is thus often necessary to integrate two or more connector units ("sockets") for similar or different purposes into respective devices.

Patient monitoring often requires delicate and sensitive electronics, which renders respective circuitry somewhat prone to ESD (Electrostatic discharge) occurrences. The same holds for EMC (Electromagnetic compatibility) occurrences, at least in certain embodiments. Since information provided by patient monitoring devices is often crucial to the treatment and well-being of the monitored patient, functional failures and defects might have severe consequences. Therefore, certain regulatory requirements exist that define the required level of ESD compliance (and further design and functional specifics).

A general design goal also in the field of medical devices is miniaturization, in particular in the field of mobile/wearable patient monitoring devices. However, miniaturization may not jeopardize the above-mentioned ESD compatibility and compliance with other functional requirements, even though a higher packing density of connectors and respective electronics as such would in theory increase the risk of unintended electrostatic discharges or, more generally, unintended shorts.

Another design goal in the field of medical devices and patient monitors is sustainability. Sustainability may involve an easy-to-repair and service-friendly design. Sustainability may also involve a reduction of the overall part diversity that is necessary to provide a certain minimum set of different products (e.g., patient monitor portfolio). This may also include a forward compatibility and a backward compatibility of at least some of the components involved.

### SUMMARY OF THE INVENTION

In view of the above, it is an object of the present disclosure to provide a connector interface for a patient monitoring module that integrates one or more connector units for connecting patient probes, sensors, consumables and other equipment. It is desirable to provide a connector interface that exhibits a repair-friendly and service-friendly design. It is desirable to present a connector interface that exhibits a modular design (due to advanced customization). Furthermore, it is desirable to present a connector interface that enjoys the most possible freedom of design while preferably utilizing standard connector components. Furthermore, it is desirable to provide a connector interface that is appropriately configured to comply with ESD requirements and other functional constraints. Likewise, it is further desirable to present a patient monitoring module that is equipped with such an interface.

In a first aspect of the present disclosure, there is presented a connector interface for a patient monitoring module, the connector interface comprising:
- a case part having an outer side and an inner side,
- at least one connector opening formed in the case part and extending between the outer side and the inner side,
- at least one connector unit that is arranged at the at least one connector opening,
   wherein the connector unit is accessible through the connector opening from the outer side in an engagement direction, and
- a mounting piece having at least one seat for the at least one connector unit,
wherein the mounting piece is attached to the case part at the inner side thereof, thereby securing the connector unit in a mounting position with respect to the case part.

This aspect is based on the insight that by applying a separate mounting piece, uniform connector units having uniform design, shape, and dimensions can be combined to form the connector interface. In other words, a variety of connector interfaces can be formed based on a set of standard connector units that are mounted to and secured with respect to a respective case part by a respectively adapted mounting piece.

For instance, when a new generation of a patient monitoring module is developed, typically also the case part (housing) thereof, is also redesigned. In accordance with the present disclosure, the standard connector units can still be used in such a case, whereas a mounting piece is used to keep the connector units in place with respect to the case part. The case part may be a connector panel of the patient monitoring module. The mounting piece may be one of a mounting part, mounting frame and/or a mounting bracket. Typically, a plurality of connector units is combined to form and accomplish the connector interface of the patient monitoring module. Hence, in exemplary embodiments, a single mounting piece is used as a mounting aid for a plurality of connector units.

By contrast, with conventional connector interfaces for patient monitoring modules, a specific type of monitoring module requires a specific, individual rigid connector block unit sub-assembly that includes a number of connector units that cannot be detached from one another for maintenance/repair purposes. Further, with such conventional arrangement, the variety of parts that has to be kept in stock for maintenance/repair purposes is rather high as the connector block unit sub-assembly is typically specifically adapted to the design of the respective case part. Likewise, even when a uniform case part is utilized for different types (configurations) of monitoring modules that have a different connector configuration, a respective number of (rather costly) sub-assemblies have to be kept in stock, due to the lack of interchangeability on the level of the individual connectors. In addition, field service and customer repairs are more complicated through complex and costly parts inventory management during the service lifetime of the devices. In accordance with the above and other aspects, the present disclosure addresses at least some of these drawbacks.

As used herein, connector units can be used to connect sensors, cables, lines, leads, electrodes, consumables and other equipment that enables/facilitates patient monitoring to the patient monitoring module. This may involve equipment for blood pressure entering, respiration rate monitoring, ECG monitoring, CO2 monitoring, blood oxygen saturation (SpO2) monitoring and the like.

When standard connector units can easily be combined to form a plurality (i.e., two or more) of connector units that are part of a connector interface, the connector interface is suitably designed to facilitate repair/maintenance purposes. Further, the number of different parts to be kept in stock for repair/maintenance purposes can be significantly reduced. In an exemplary embodiment, providing a redesigned connector interface merely requires new parts on the level of the case part and on the level of the mounting piece, but not on the level of the connector units. That is, the parts that are crucial for the function of the patient monitoring module do not have to be redesigned. Individual connector units can be combined to form a row of connector units. At least some of the connector units can be arranged in a neighboring relationship, i.e., next to one another.

As used herein, the engagement direction coincides with an engagement axis that is basically orthogonal to an opening area provided by the connector opening or the front face of the connector unit. In certain embodiments, the connector interface has a plurality of connector openings, each being assigned to a respective connector unit, wherein the engagement directions for the connector units are basically parallel to one another.

In certain embodiments, the connector units are individually replaceable for retrofit, repair and/or maintenance purposes.

In certain embodiments, the case part is an integral part of an overall housing of the patient monitoring module. In certain embodiments, the case part is a connector panel that is arranged to be mounted to a frame and/or an overall housing of the patient monitoring module.

In an exemplary embodiment, the connector interface comprises two or more neighboring connector units that are arranged next to one another, wherein two or more connector openings are formed in the case part, wherein each of the two or more connector units is arranged at one of the two or more connector openings, wherein the mounting piece has two or more seats for the two or more connector units, and wherein the mounting piece secures the two or more connector units in a respective mounting position with respect to the case part.

In accordance with this embodiment, the mounting piece embraces/combines the two or more connector units, fastens and secures them (directly or mediately) at the case part. This may include embodiments in which the individual connector units would simply fall off the case part if the mounting piece was not provided. Transferring the mounting/securing function to the mounting piece may have the effect that on the level of the individual connector units, no individual fastening features (e.g., screw holes, etc.) have to be provided. This is not to be understood in a limiting sense. Further, such scenario does not exclude that positioning/orientation features are provided on the level of the individual connector unit. However, in exemplary embodiments, the mounting piece serves as a mounting bracket/frame that is put onto a plurality of connector units that are arranged in a respective mounting position to fasten and secure them mediately to the case part. That is, in certain embodiments, the mounting piece is screwed to the case part, whereas the individual connector units are not screwed to the case part, but for instance slightly biased by the mounting piece against the case part.

In another exemplary embodiment, a divider is arranged on a rear side of the mounting piece, the divider forming separate compartments for separate electronics modules that are coupled to the connector units, wherein partition walls of the divider are spaced from one another by an offset that is adapted to a width of associated neighboring connector units.

By way of example, the connector interface includes at least one connector row that is formed of a plurality of connector units that are arranged adjacent to one another.

With patient monitoring systems and modules, several regulatory requirements have to be observed. This may include the provision of minimum creepage paths and/or minimum insulation clearances to avoid electrostatic discharge (ESD) during operation and/or the module's service life. Similar provisions may apply to electromagnetic compatibility (EMC), etc.

In view of the compact connector package that can be achieved by including a row of adjacent connector units, the divider may define and separate respective compartments that are associated with respective ones of the connector units. Hence, even though a rather tight package of connector units can be provided, functional requirements, such as ESD compliance and/or EMC compliance are maintained.

As used herein, electronics may refer to individual printed circuit boards (PCBs) for the connector units, the PCBs being equipped with respective circuitry.

In addition, as the divider provides respective compartments, a defined space for electronics associated with the respective connector units is provided. In certain embodiments, the patient monitoring module has a modular design as regards the respective measurements. That is, a measurement value that is processed based on a signal that is delivered through an individual connector unit is (at least to some extent) processed using electronics/circuitry that is provided only for this particular purpose, and that is embodied by an individual PCB/electronics module.

Hence, the modular approach applies not only to the level of the connectors, but also to the data processing level, at least in certain embodiments. Hence, an individual measurement (e.g., temperature, respiration rate, ECG, arterial oxygen saturation (SpO2) and the like) is performed by a functional module that includes a connector unit and an associated electronics module.

In certain embodiments, the divider is arranged at the rear side of the mounting piece that is facing away from the case part. In certain embodiments, the respective compartments provided by the divider may have a cross-sectional shape that corresponds to a projection of the cross-sectional area of the connector units to the rear side.

In certain embodiments, the dimensions of a connector unit also determine the space that is provided for respective electronics modules. By way of example, when two or more connector units are arranged in a row adjacent to one another, the respective width or spacing therebetween defines the space that can be occupied by respective electronics. This space can be further defined by the divider providing respective compartments (separated from one another by the partition walls). This may result in individual PCBs for the connector units, the PCBs having a narrow and elongated design that is adapted to the width of the connector unit and the room provided at the rear side of the mounting piece. The partition walls ensure the sufficient length of the creepage paths between neighboring connector units/electronics modules.

In another exemplary embodiment, the at least one connector unit comprises a connector housing, wherein the mounting piece urges the connector housing against the case part's interior. In this way, the connector unit is mediately mounted and secured to the case part. As the mounting piece is fastened to the case part, the mounting piece may push the individual connector units into respective mounting seats at the case part.

In certain embodiments, the connector housing of the connector unit serves as a support structure that contains a contact insert that is arranged therein.

In certain embodiments, the mounting piece contains push features such as push bars and the like that impact on a flange of the connector housing to push the same against the case part.

In certain embodiments, the connector housing is resiliently and/or flexibly held by the mounting piece in its mounting position with respect to the case part.

In certain embodiments, two or more distinct connector units (e.g., for different measurements) share a common connector housing, wherein different contact inserts accommodated therein define the specific parameter type of the connector unit.

In another exemplary embodiment, mounting features are formed at the connector housing and the case part, which mounting features define a correct mounting orientation for the connector housing. In such case, mistake-proof assembly (cf. poka-yoke) can be provided. For instance, the mounting features may define a respective rotation orientation of the connector housing with respect to the case part.

Further, the mounting features may increase the connector unit's ability to withstand excessive torque and/or other mechanical peak loads during operation, e.g., when plugging in or unplugging respective connector plugs.

In another exemplary embodiment, the connector housing comprises a frontal collar and a flange, wherein the frontal collar protrudes beyond the flange towards the outer side.

In certain embodiments, the frontal collar of the connector housing contacts the case part in the mounted state of the connector unit. By way of example, the frontal collar engages a corresponding recess at the inner side of the case part. In certain embodiments, the frontal collar is arranged as a hollow stud that surrounds the contact insert.
The flange may be referred to as abutment flange that is contacted by the mounting piece.

In certain embodiments, the connector housing has a basically tubular shape with the flange forming a protruding outer contour onto which the mounting piece acts for securing the mounting position of the connector unit.

In another exemplary embodiment, at least one of the frontal collar and the flange comprises a positioning aid that is configured to cooperate with a corresponding mating portion formed at the case part. In this way, the connector unit and the case part may cooperate to define a desired orientation of a counterpart that is inserted in the connector interface. This may prevent from improper use during operation of the patient monitoring module.

In certain embodiments, the positioning aid is arranged as at least one of a positioning recess and a positioning projection. By way of example, a positioning tab may be formed as an axial and/or radial extension of the frontal collar, wherein a corresponding recess is formed at the connector opening of the case part. By way of example, the frontal collar may provide a positioning aid in the form of a flat side area that is adapted to a corresponding flat portion of the connector opening of the case part. In either case, mistake-proof mounting (cf. poka-yoke) of the connector unit is facilitated.

It is to be noted in this context that in accordance with some aspects of the present disclosure, the primary function of the mounting piece is securing and fastening the connector units at the case part, whereas the correct position and orientation of the individual connector units is provided by a cooperation of the case part and the connector units (particularly the connector housings). In this way, the functional separation simplifies the design of the mounting piece.

In another exemplary embodiment, at least one connector unit further comprises a conductive sheet, wherein the flange comprises a support portion to which the conductive sheet is attached. Accordingly, the flange does not only serve mounting purposes, but also as a carrier for the conductive sheet. The conductive sheet enables the dissipation of potential electrostatic charges.

In another exemplary embodiment, and conductive sheet comprises at least one side contact portion that is configured to contact a side contact portion of a conductive sheet of a neighboring connector unit, when two or more connector units are disposed at the same case part next to one another.

When two or more connector units are provided with a conductive sheet, their contacted sheets can be automatically connected when the connector units are placed in a corresponding adjacent relationship during assembly. In this way, two or more conductive sheets can be connected (and eventually grounded via additional conductors). The individual connector units may thus be equipped with ESD protection features on the individual level, whereas an arrangement in a row automatically couples the individual ESD protection features.

Two or more connector units that are arranged next to one another may form a row (linear pattern) of connector units. It is also possible to form two or more rows of connector units, thereby forming an array of connector units that are arranged in rows and columns. In either case, conductive sheets may be provided on the level of the connector units that become interconnected when arranging the connector units next to one another.

The conductive sheets typically have two opposite side contact portions (e.g., left side and right side, or top side and bottom side). Hence, the connector unit can be conductively connected to two neighboring connector units. The conductive sheets are typically made of conductive metal sheets/strips.

In another exemplary embodiment, a connecting bar is arranged at the mounting piece, wherein the connecting bar comprises two or more resilient receptacles, and wherein the conductive sheet contacts one of the two or more resilient receptacles in the mounted state for dissipating electrostatic charges. In this way, the conductive sheets of neighboring connector units are not directly connected but mediately via the connecting bar for dissipating electrostatic charges. The connecting bar may also be referred to as busbar.

The connecting bar interconnects the conductive sheets of a plurality of connector units, which each are conductively connected to a respective resilient receptacle of the connecting bar. The two or more resilient receptacles may be composed of two opposite resilient arms that can accommodate therebetween a protruding contact portion of the conductive sheets. In this way, the conductive sheets are arranged to engage the resilient receptacles of the connecting bar, while the resilient design is tolerant to geometrical and/or positional deviations (e.g., caused by shock impact during monitor use, vibrations, etc.).

The connecting bar may be mounted to the mounting piece, in particular at the side thereof that faces the outer side. Fastening the connecting bar to the mounting piece may involve engaging at least one mounting recess at the connecting bar with mounting protrusions formed at the mounting piece. The mounting protrusions may be thermally deformed (e.g., via heat/hot stamping) to secure the connecting bar in a loss-proof manner to the mounting piece.

In certain embodiments, the connector units may also serve as parameter interfaces among various applications (e.g., extension modules for other patient monitoring modules) and/or be incorporated into future generation patient monitoring modules and related products. The adopted approach is sustainable and/or future-proof, as with future designs the connector units may still be used, while an adaption to new (case) designs is provided by the respective mounting piece.

In certain embodiments, the support portion is an integral part of the flange. In certain embodiments, the side contact portions embrace the support portion. In this way, the side contact portions do not only serve (electrically) conductive purposes, but also as an attachment feature for mounting the conductive sheet to the support portion of the flange.

In another exemplary embodiment, at least one mounting protrusion is formed at the support portion, wherein the at least one mounting protrusion engages the conductive sheet and a flex foil that is contacted by the conductive sheet. For instance, the conductive sheet is provided with mounting holes through which corresponding mounting pins extend to achieve a positive locking of the conductive sheet at the support portion.

In another exemplary embodiment, the at least one mounting protrusion also engages a flex foil that connects the contact insert of the connector unit and a rear electronics module (e.g., PCB assembly).

In addition, in certain embodiments, the at least one mounting protrusion is thermally deformed (e.g., via heat/hot stamping) to secure the conductive sheet in a loss-proof manner. Hence, attaching the conductive sheet and also a flex foil to the support portion may involve a pin and hole configuration and a thermal deformation of the pin.

In another exemplary embodiment, the at least one connector unit comprises a contact insert, wherein the contact insert has a front contact portion that is arranged to be contacted by a mating connector plug through the connector opening.

The contact insert is arranged in the connector housing. By way of example, the front contact portion includes front contacts and thus forms a connector socket for a plug that is to be inserted in the connector unit. In view of the front contact portion, the contact insert may be arranged to be compliant with standards for medical measurement equipment. In certain embodiments, the contact insert serves as a support/carrier for conductors that connect the outer side and the inner side. In certain embodiments, the contact insert includes a fluid passage, e.g., a gas passage.

In certain embodiments, the connector unit provides a socket that is configured to receive a respective plug. It is to be noted that the terms "socket" and "plug" do not exclude that for instance male contacts are formed at the socket and female contacts are formed at the plug.

The contact may include signal contacts and/or power contacts. Signals may involve analog signals, digital signals, measurement data, communication data, etc.

In another exemplary embodiment, the contact insert has a rear contact portion, wherein a flex foil is attached to the rear contact portion, wherein the flex foil contacts back contacts of the connector unit, and wherein the flex foil is arranged to connect the back contacts of the connector unit to an electronics module that is operatively coupled to the connector unit.

In other words, the connector unit with the contact insert may serve as an interface between the exterior (environment) and the interior of the patient monitoring module.

In certain embodiments, the flex foil is permanently (non-releasably) contacting the back contacts. The flex foil may include one or more conductive paths that are formed on a flexible sheet. The flex foil may provide a conductive connection between the contact insert and a rear electronics module.

In another exemplary embodiment, the flex foil contacts the conductive sheet at the support portion of the flange. In this way, electrostatic charges can be dissipated via the conductive sheet to protect sensitive electronics inside the patient monitor device. Also in this way, the connector interface may have an ESD compliant design.

In another exemplary embodiment, the connector housing has a peripheral wall that surrounds the rear contact portion, wherein the rear contact portion is encapsulated within the peripheral wall. In this way, hermetic sealing between the outer side/exterior and the inner side/interior can be provided, leakage through the connector unit and in particular through the contact insert thereof, can be minimized or entirely prevented.

In certain embodiments, the rear contact portion is sealed with potting compound that is provided inside the peripheral wall of the connector housing.

In another exemplary embodiment, the connector opening has a centering recess at the inner side and a mouth at the outer side, wherein the centering recess is adapted to a centering contour of the connector unit, and wherein the mouth forms an access at the outer side having an opening area that is smaller than a cross-sectional area of the centering recess.

In other words, the centering contour and the mouth of one and the same connector opening may have different cross-sections, as seen along an axis that is orthogonal to a front face of the connector unit. As seen from the outer side, the centering contour is formed behind the mouth in the case part. The mouth is facing the outer side. The connector unit can engage the centering recess with the centering contour that is formed at the frontal collar of the connector housing.

In another exemplary embodiment, a circumferential gasket is arranged between the case part and the connector unit which seals an abutment position of the connector unit at the connector opening. By way of example, the circumferential gasket is arranged as an O-ring and/or a ring gasket. In certain embodiments, the circumferential gasket is a molded or otherwise specifically shaped gasket that is adapted to the mating/joining contours of the connector units and the connector openings at the case part.

In certain embodiments, the circumferential gasket is a (primarily) radially sealing gasket. In certain embodiments, the circumferential gasket is a (primarily) axially sealing gasket. In certain embodiments, the circumferential gasket is a gasket that seals axially and radially. In certain embodiments, the circumferential gasket is an O-ring and/or a ring gasket that is mounted to the frontal collar of the connector housing to be urged between the frontal collar and the centering recess of the connector opening.

In certain embodiments, the circumferential gasket is a flat gasket that is mounted to the flange of the connector housing. This may involve a mounting to the flange via snap-in studs that are formed at the circumferential gasket and extend through mounting holes in the flange towards the inner side to lock the circumferential gasket to the flange.

In certain embodiments, the circumferential gasket is arranged between the centering recess of the connector opening and the centering contour of the connector housing. In this way, a tight fit of the connector unit and the connector opening can be achieved. In certain embodiments, the centering contour and the centering recess are each cylindrical. The centering recess provides a seat for the frontal collar of the connector housing.

It is to be noted in this context that there may be different design features that ensure proper positioning and orientation of the connector units and the connector openings of the case part. Such assembling design features may include the centering recess at the case part plus a corresponding centering contour at the connector housing. The centering recess and the centering contour each may have a circular/annular shape so that the axes of the connector unit with respect to the case part can be aligned.

Such assembling design features may also include mounting features at the flange of the connector housing and corresponding mounting features at the case part. This may include mounting depressions and corresponding mounting recesses that are arranged to engage one another. In this way, a rotational orientation between the connector unit and the connector opening may be realized. Further, the mounting features may be configured to withstand torque loads and the like.

The assembling design features may also include positioning aids at the connector housing and corresponding mating portions at the case part. Also in this way, a desired rotational orientation (i.e., intended coding) between the connector housing and the connector opening of the case part may be achieved. This may include embodiments where the positioning aids and the mating portions are adapted to a design of a connector plug that is configured to be plugged into a respective connector unit. Overall, a poka-yoke design may be provided that fosters mistake-proof assembling.

In yet another aspect of the present disclosure there is presented a patient monitoring module comprising a connector interface in accordance with at least one aspect or embodiment as disclosed herein.

The connector interface may form part of a patient monitoring module. The patient monitoring module may be a stationary patient monitoring module, e.g., a bedside patient monitor. The patient monitoring module may also be a mobile/portable patient monitoring module, e.g., an extension module that can be used to extend the functional scope of a stationary patient monitoring module. A stationary patient monitoring module may be used as a host/basis for extension models. Extension models may be of the stationary type and/or of the portable type.

There may be many embodiments of the patient monitoring module. The patient monitoring module may be a stationary module, a portable module, an extension module, etc. The patient monitoring module includes a connector interface that enables connecting one or more sensors and consumables, probes, signal cables, etc. to the patient monitoring module, thereby providing patient information that is to be processed by the patient monitoring module and/or relayed to other patient monitoring equipment.

### BRIEF DESCRIPTION OF THE DRAWINGS

These and other aspects of the disclosure will be apparent from and elucidated with reference to the embodiments described hereinafter. In the following drawings
Fig. 1 shows a perspective view of a patient monitoring module having a connector interface;
Fig. 2 shows a perspective front view of a connector interface for a patient monitoring module;
Fig. 3 shows a partially exploded view of the connector interface of Fig. 2;
Fig. 4 shows a partially exploded rear view of the connector interface of Figs. 2 and 3;
Fig. 5 shows another view of the arrangement of Fig. 4, wherein components are omitted for illustrative purposes;
Fig. 6 shows another perspective rear view of the connector interface of Figs. 2-5, wherein a mounting piece secures connector units in a mounting position at a case part;
Fig. 7 shows a partially exploded view of the connector interface of Fig. 6;
Fig. 8 shows a partially exploded frontal view of the connector interface of Fig. 7;
Fig. 9 shows an enlarged partially cross-sectional perspective frontal view of a connector unit that is mounted to a connector opening of a case part;
Fig. 10 shows an enlarged partially cross-sectional perspective rear view of the arrangement of Fig. 9;
Fig. 11 shows a perspective frontal view of a connector unit in an unmounted state;
Fig. 12 shows a perspective rear view of the connector unit of Fig. 11;
Fig. 13 shows a perspective exploded rear view of the connector unit of Figs. 11 and 12;
Fig. 14 shows a perspective rear view of a connector row that is composed of neighboring connector units;
Fig. 15 shows a perspective exploded frontal view of the connector unit of Figs. 11-13;
Fig. 16 shows a perspective frontal view of a connector row that is composed of neighboring connector units;
Fig. 17 shows a further perspective view of the arrangement of Fig. 16, where gaskets are shown in a detached state;
Fig. 18 shows a partial top view of a mounting piece and a plurality of connector units that are shown in a partially detached state;
Fig. 19 shows a perspective frontal view of the arrangement of Fig. 18; and
Fig. 20 shows a further perspective frontal view of the arrangement of Fig. 19 in a partially exploded state.

### DETAILED DESCRIPTION OF THE INVENTION

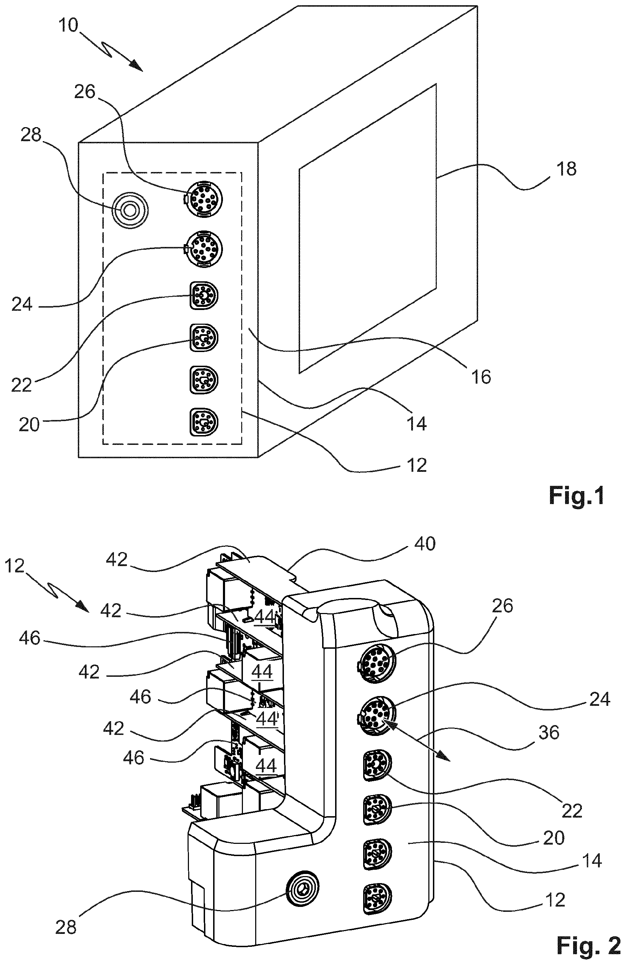

Fig. 1 shows a schematic perspective view of a patient monitoring module 10. The patient monitoring module 10 of Fig. 1 represents a variety of respective patient monitoring modules, which include stationary patient monitoring modules, mobile monitoring modules, and so-called extension models that can be connected to other patient monitoring equipment.

The module 10 comprises a connector interface 12. The connector interface 12 is formed on/in a case part 14, which may be arranged as a connector panel 16. The connector interface 12 provides an interface between the exterior and the interior of the module 10. Information, signals and data representing a patient's health condition that can be provided to the module 10 via the connector interface 12. The case part 14 may be an integral component of an overall housing of the module 10. The case part 14 may also be separable from the overall housing.

In the exemplary embodiment of Fig. 1, the module 10 has an integrated display 18 that is configured to display health information about the patient. In certain embodiments, the display 18 may be a touch-sensitive display. Consequently, the display 18 may also be used as an input for operating the module 10. Other control elements may also be provided, at least in certain embodiments.

A plurality of connector units 20, 22, 24, 26, 28 may be provided at the connector interface 12. The connector units 20, 22, 24, 26, 28 may also be referred to as sockets. The connector units 20, 22, 24, 26 may include conductive contacts. The connector unit 28 may include a fluid passage, in particular for gaseous fluids. The connector units 20, 22, 24, 26, 28 may be used to couple respective plugs to the module 10. In this way, the module 10 can be supplied with health information about the patient, for instance including heart rate, respiration rate, ECG data, arterial oxygen saturation data (SpO2), carbon dioxide (CO₂) measurements and the like. The foregoing list is not to be understood in a limiting manner.

Individual reference numerals 20, 22, 24, 26, 28 designate different types of connector units in the exemplary embodiments. However, the connector interface 12 may include two or more connector units of the same type, refer to reference numeral 20, for instance. The connector units 20, 22, 24, 26, 28 may be of a specific design for a specific measurement purpose. The connector units 20, 22, 24, 26, 28 may also be of a standard design, whereas still different measurement tasks are possible. Even with different and distinct purposes, the connector units 20, 22, 24, 26, 28 may share at least some common design features, in particular in regard of the mounting to the case part 14.

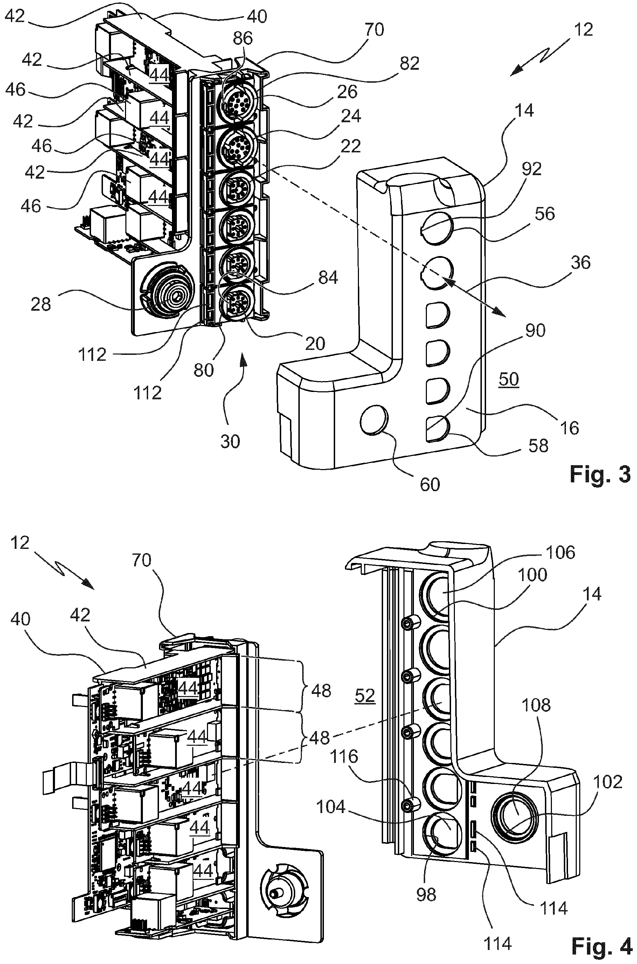

Fig. 2 shows a perspective frontal view of a connector interface 12 that may form part of a patient monitoring module 10. Fig. 3 shows a corresponding partially exploded view. Fig. 4 shows a corresponding partially exploded rear view. The respective path of the exploded view of Figs. 3 and 4 is parallel to the engagement direction 36. As already discussed in connection with Fig. 1, the interface comprises a case part 14 that may be implemented as a connector panel in the exemplary embodiment.

Several connector units 20, 22, 24, 26, 28 are provided at the connector interface 12. Cables, lines, leads, hoses etc. may be fed with their plugs in an engagement direction 36 towards a respective socket provided by one of the connector units 20, 22, 24, 26, 28. In this way, sensory accessories may be plugged into the connector units 20, 22, 24, 26, 28 to couple to respective circuitry of the patient monitoring module 10.

In the exemplary embodiment of Figs. 2-4, a divider 40 is provided on a rear side of the respective connector units 20, 22, 24, 26. The divider 40 includes partition walls 42 extending in the engagement direction 36, thereby forming individual compartments 44 that may be assigned to respective ones of the connector units 20, 22, 24, 26 in a one-to-one allocation. The compartments accommodate electronics modules 46 that are functionally coupled to a respective connector unit 20, 22, 24, 26. In this way, also on the level of the data processing for the individual connector units 20, 22, 24, 26, a modular approach is possible, at least in certain embodiments. An offset between neighboring partition walls 42 is indicated in Fig. 4 by 48. The offset 48 corresponds to a width of a respectively assigned connector unit 20, 22, 24, 26.

Fig. 3 illustrates an outer side 50 of the case part 14. Fig 4 illustrates an inner (rear) side 52 of the case part 14. The outer side 50 corresponds to the exterior of the module 10 (refer to Fig. 1). The inner side 52 corresponds to the interior of the module 10.

As can be best seen in Fig. 3, connector openings 56, 58, 60 are formed in the case part 14 to provide access to the respective connector units 20, 22, 24, 26, 28 that are placed there behind, refer also to Fig. 2. Some of the connector openings 56, 58, 60 may be of the same design, depending on the type of the connector unit 20, 22, 24, 26, 28 that is to be mounted there.

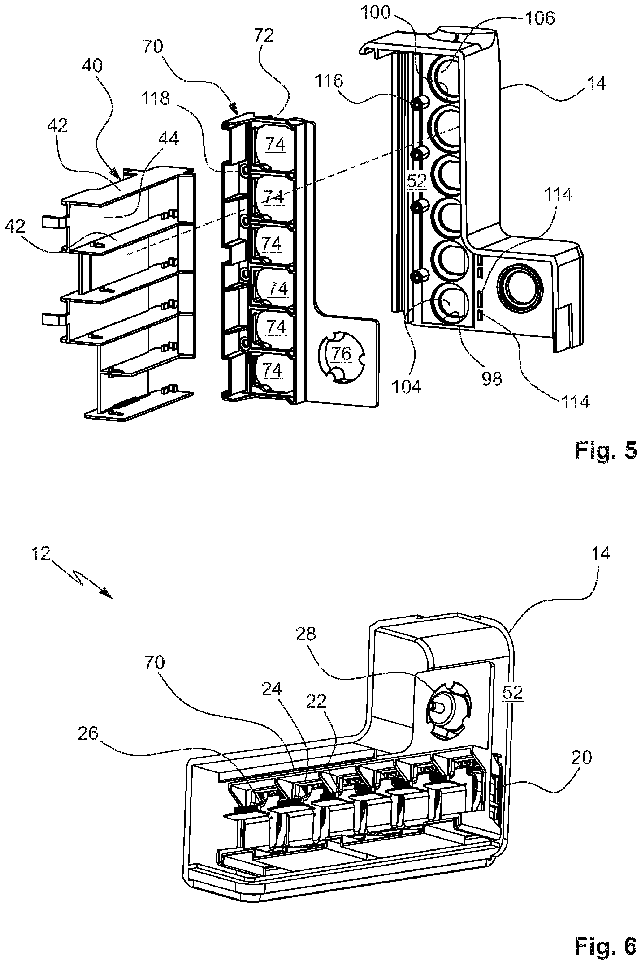

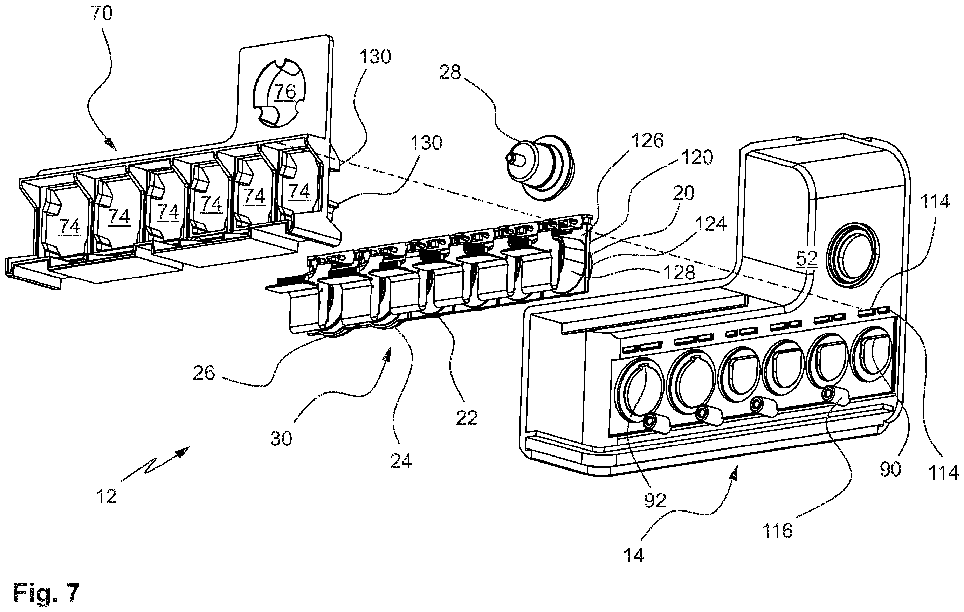

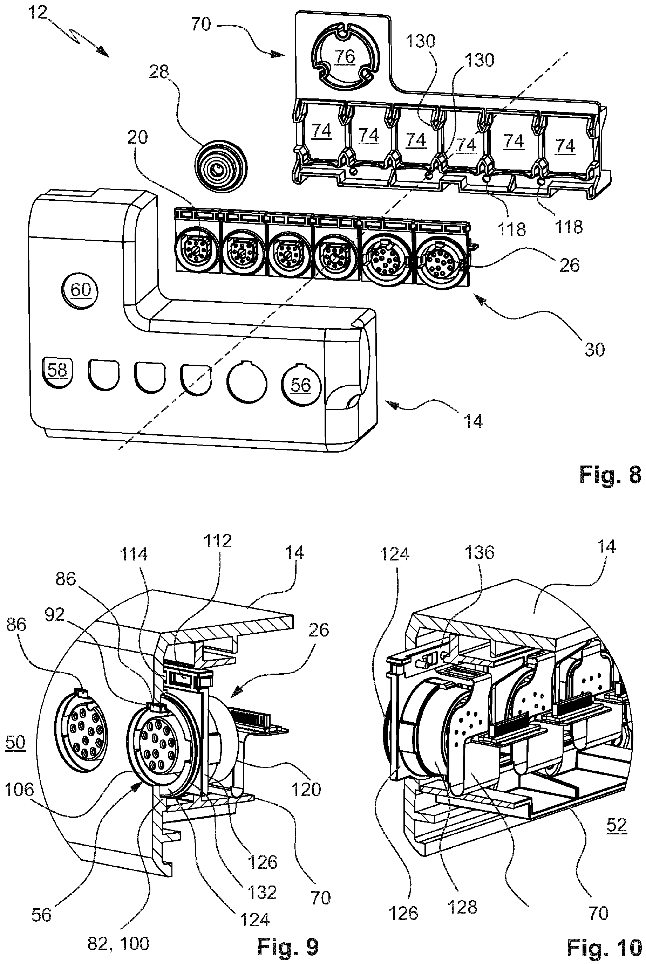

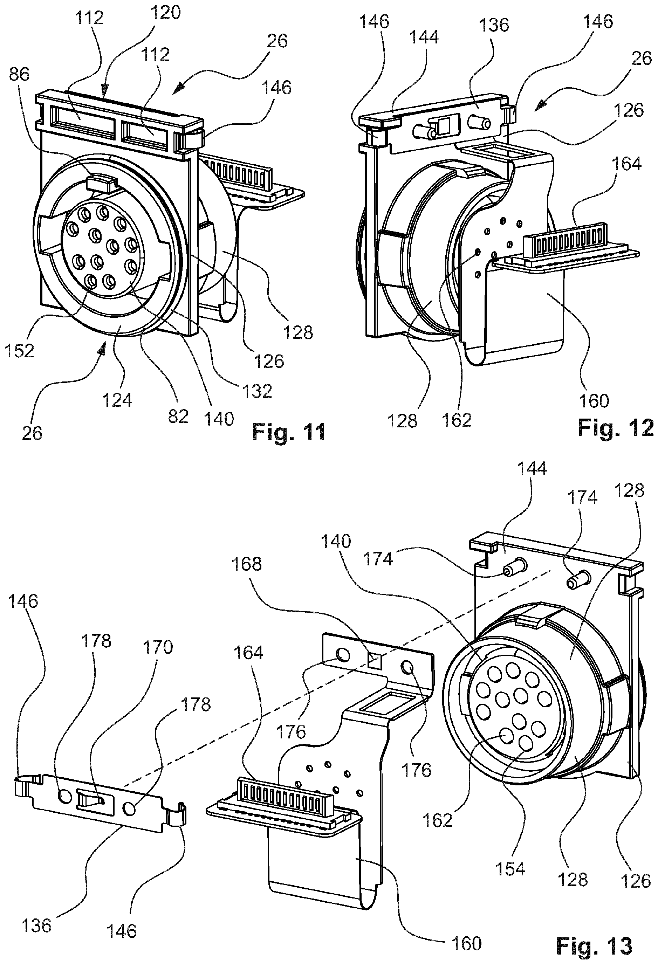

A mounting piece 70 is provided as a mounting aid that secures the connector units 20, 22, 24, 26, 28 in their mounted state at the inner side 52 of the case part 14. It is to be noted in this context that the connector units 20, 22, 24, 26, 28 may be individual components, refer also to Figs. 11-13 in this context. Hence, the arrangement of the connector units 20, 22, 24, 26 in a row 30 as shown in Fig. 3 (see also Figs. 7 and 8) is not inherently stable, the connector units 20, 22, 24, 26 may simply fall apart without being mounted and secured through the mounting piece 70. As indicated in Fig. 5, the mounting piece 70 may be arranged as a mounting frame 72 providing seats 74, 76 for the individual connector units 20, 22, 24, 26, 28. The mounting piece 70 is arranged to push the connector units 20, 22, 24, 26, 28 against the case part 14 to secure and keep them in place at the respective connector openings 56, 58, 60.

The orientation and position of the connector units 20, 22, 24, 26, 28 in the mounted state (see Fig. 2) may be defined by a respective centering contour 80, 82 and a positioning aid 84, 86. In the exemplary embodiment of Fig. 3, the centering contour 80, 82 and the positioning aid 84, 86 are formed at a frontal end of the connector units 20, 22, 24, 26. Mating portions 90, 92 that correspond to the positioning aids 84, 86 are formed at the connector openings 56, 58. The positioning aids 84, 86 and the corresponding mating portions 90, 92 define a rotation orientation of a connector plug that engages a respective one of the connector units 20, 22, 24, 26 in the mounted state.

As illustrated in Fig. 4, the connector openings 56, 58, 60 may also include a centering recess 98, 100, 102 that is adapted to the respective centering contour 80, 82 of the connector units 20, 22, 24, 26, 28. In the exemplary embodiment, the centering recesses 98, 100, 102 and the centering contours 80, 82 are both cylindrical so that a proper alignment and a tight fit of the connector units 20, 22, 24, 26, 28 at the connector openings 56, 58, 60 is enabled. In addition to the centering recesses 98, 100, 102, the connector openings 56, 58, 60 further include mouths 104, 106, 108 at the outer side of the case part 14. The mouths 104, 106, 108 define a respective entrance for (male) connector counterparts (e.g., connector plugs).

To ensure a proper orientation of the connector units 20, 22, 24, 26 at the case part 14, additional mounting features 112, 114 are provided. The mounting features 112, 114 may be referred to as coding features. In the exemplary embodiments, on the level of the connector units 20, 22, 24, 26, depressed mounting features 112 are formed that match corresponding elevated mounting features 114 that are formed on the inner side 52 of the case part 14. The case part 14 may also be provided with depressed mounting features, while the connector units 20, 22, 24, 26 may be provided with elevated mounting features. The mounting features 112, 114 are spaced from respective centering contours 80, 82 and centering recesses 98, 100, 102 so that bringing the mounting features 112, 114 into alignment and mutual engagement results in a proper rotation orientation of the connector units 20, 22, 24, 26 in the mounted state at the case part 14.

At least in certain embodiments, it is not the primary goal of the mounting piece 70 to place the connector units 20, 22, 24, 26, 28 in a proper mounting position and orientation. This is performed by a cooperation between the mounting features of the connector units 20, 22, 24, 26, 28 at the case part. Rather, the primary goal of the mounting piece 70 is to push the connector units 20, 22, 24, 26, 28 against the case part 14 and to keep them in place. The mounting piece 70 is a fastening aid for the mounting of the connector units 20, 22, 24, 26, 28. The mounting piece 70 is arranged to be fastened to the case part 14, e.g., by means of a screw fastening using mounting bosses 116 formed at the case part 14 and corresponding mounting holes 118 formed at the mounting piece 70, see also Fig. 5.

The mounting piece 70 is a component that is specifically adapted to the design of the case part 14. By contrast, the connector units 20, 22, 24, 26, 28 do not necessarily have to be specifically designed being conform to the individual overall design of the case part 14. Rather, the connector openings 56, 58, 60 have a standard design to provide seats for the individual connector units 20, 22, 24, 26, 28. The pattern of connector units 20, 22, 24, 26, 28 that is provided at the connector interface 12 can be composed of standard components whose placement and arrangement is defined by the neighboring components (having a specific design), primarily by the case part 14 and the mounting piece 70.

Figs. 4 and 5 illustrate that the divider 40 forming the compartments 42 is arranged on a rear side of the mounting piece 70 to provide sufficient space for electronics modules 46 that connect to the connector units 20, 22, 24, 26. The divider 40 enhances the ESD compliance of the connector interface 12 and a respectively equipped patient monitoring module 10.

Fig. 6 is a perspective rear view of the connector interface 12, with the divider 40 and respective circuitry being omitted. The connector units 20, 22, 24, 26, 28 are mounted to the case part 14 with the aid of the mounting piece 70. In the exemplary embodiment, the connector units 20, 22, 24, 26, 28 are not directly but mediately fastened to the case part 14.

Fig. 7 further exemplifies the mounting concept that makes use of the mounting piece 70. The mounting piece 70 has seats 74, 76 for the connector units 20, 22, 24, 26, 28. In certain embodiments, the seats 74, 76 for the connector units 20, 22, 24, 26, 28 provide a loose fit in terms of positioning and aligning the connector units 20, 22, 24, 26, 28 in the centering recesses 98, 100, 102 at the case part 14. The same may apply to the rotation orientation that is ensured by the cooperation between the mounting features 112, 114. The primary purpose of the mounting piece 70 is to secure the connector units 20, 22, 24, 26, 28 in the mounted state. Hence, the task of positioning and securing the connector units 20, 22, 24, 26, 28 is split between the mounting piece 70 and other positioning features formed between the connector units 20, 22, 24, 26, 28 and the case part 14.

The connector units 20, 22, 24, 26, 28 are provided with a respective connector housing 120, which comprises a frontal collar 124 and a flange 126. Refer also to Figs. 8-13 in this context. In the exemplary embodiment, at least the flange 126 of the connector units 20, 22, 24, 26 may have a rectangular shape (in a frontal/rear view). The mounting piece 70 contains several push bars 130 that push the flanges 126 of the connector units 20, 22, 24, 26, 28 against the case part 14. Hence, the respective frontal collar 124 may engage the centering recess 98, 100, 102 and remains in place. At the opposite side of the flange 126, a circumferential wall 128 forms part of the connector housing 120.

Partially cross-sectional views of Figs. 9 and 10 illustrate the mounted state of one of the connector units 26. Figs. 11 and 12 illustrate the connector unit 26 in a segregated (non-mounted) state. It is to be noted that some components (e.g., pins, springs and the like) have been omitted for illustrative purposes in the Figures. At least the connector units 20, 22, 24 exhibit a similar design. At the frontal collar 124 of the connector housing 120, a positioning aid 86 is formed that engages a mating portion 92 at the mouth 106 of the connector opening 56. The frontal collar 124 also forms the centering contour 82 that engages the centering recess 100 of the connector opening 56 in the mounted state. At the frontal collar 124, a circumferential gasket 132 is arranged that seals the mounted state of the connector unit 26 at the connector opening 56.

The mounting piece 70 pushes the flange 126 against the case part. The mounting features 112 are formed at the flange 126, the corresponding engaging mounting features 114 are formed at the inner side 52 of the case part 14. The circumferential wall 128 at the rear end of the connector unit 26 extends into one of the seats 74 of the mounting piece 70, see also Fig. 8.

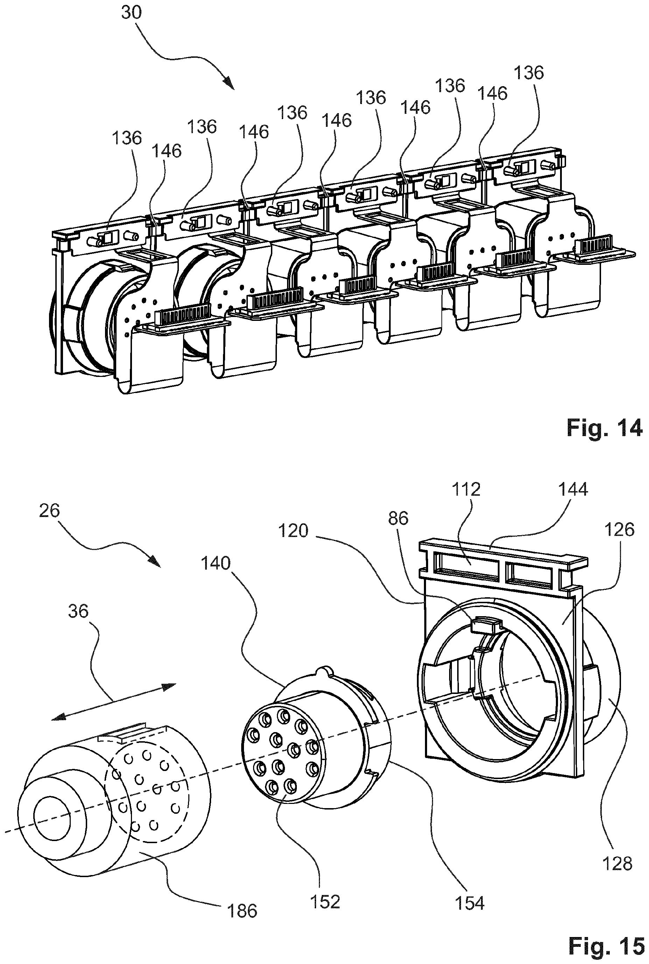

Reference is made to Figs. 11-15, further detailing the design of the connector unit 26. A contact insert 140 is accommodated in the connector housing 120, see also the partially exploded view of Fig. 15 with the contact insert 140 detached from the connector housing 120.

A conductive sheet 136 is attached to the connector housing 120, in particular to a support portion 144 formed at the flange 126. The conductive sheet 136 forms part of a discharge path for dissipating unintended electrostatic charges or, more generally unintended shorts. In the exemplary embodiment, the conductive sheet 136 has two opposite side contact portions 146 that embrace the support portion 144. When two or more connector units 20, 22, 24, 26 having conductive sheets 136 are arranged in a row 30, the side contact portions 146 of the conductive sheets 136 of neighboring connector units 20, 22, 24, 26 contact one another and therefore the conductive sheets 136 together form part of the discharge path. See in this context also Fig. 14. It is to be noted here that in Fig. 14, the connector row 30 is still composed of individual connector units 20, 22, 24, 26 that would fall apart without additional mounting features (as provided by the case part 14 and the mounting piece 70).

The contact insert 140 has a front contact portion 152 (Fig. 11) and a rear contact portion 154 (Fig. 13). Respective (conductive) contacts (e.g., metal pins, sleeves, and the like) that connect the front contact portion 152 and the rear contact portion 154 are omitted in the Figures. The rear contact portion 154 is surrounded by the peripheral wall 128. In addition, the rear contact portion 154 may be encapsulated (e.g., potted) within the peripheral wall 128.

A flex foil 160 contacts back contacts 162 at the rear contact portion 154 of the contact insert 140 and connects them to a contact bar 164 that is configured to connect to a respective electronics module 46 (Figs. 2 and 3). Hence, the flex foil 160 connects the connector units 20, 22, 24, 26 and the respective circuitry at the electronics modules 46. The flex foil 160 is also coupled to the conductive sheet 136, refer to Fig. 13. To this end, the flex foil 160 has a conductive portion 168 that is arranged to be contacted by a contact lug 170 of the conductive sheet 136. In other words, the conductive portion 168 is interposed between the support portion 144 of the flange 126 and the conductive sheet 136.

Fig. 13 also shows that mounting protrusions 174 are formed at the rear side of the flange 126 at the support portion 144. Corresponding holes 176, 178 are formed at the flex foil 160 and the conductive sheet 136. The flex foil 160 and the conductive sheet 136 may be put onto the mounting protrusions 174 with their holes 176, 178 to be positioned and kept in place after installation. In certain embodiments, the mounting protrusions 174 can be thermally deformed (not shown in Fig. 13) to secure the flex foil 160 and the conductive sheet 136 at the support portion 144 in a loss-proof manner.

Fig. 15 illustrates the connector housing 120 with the flange 126 and the contact insert 140 as separate components of the connector unit 26. Further, reference numeral 186 indicates a mating counterpart (connector plug) that is adapted to the front contact portion 152 and the overall geometry of the connector unit 26 with the connector housing 120 and the contact insert 140. The drafted/sketched male connector counterpart 186 (e.g., connector plug) can be fed to and plugged in the connector unit 26 in the engagement direction 36.

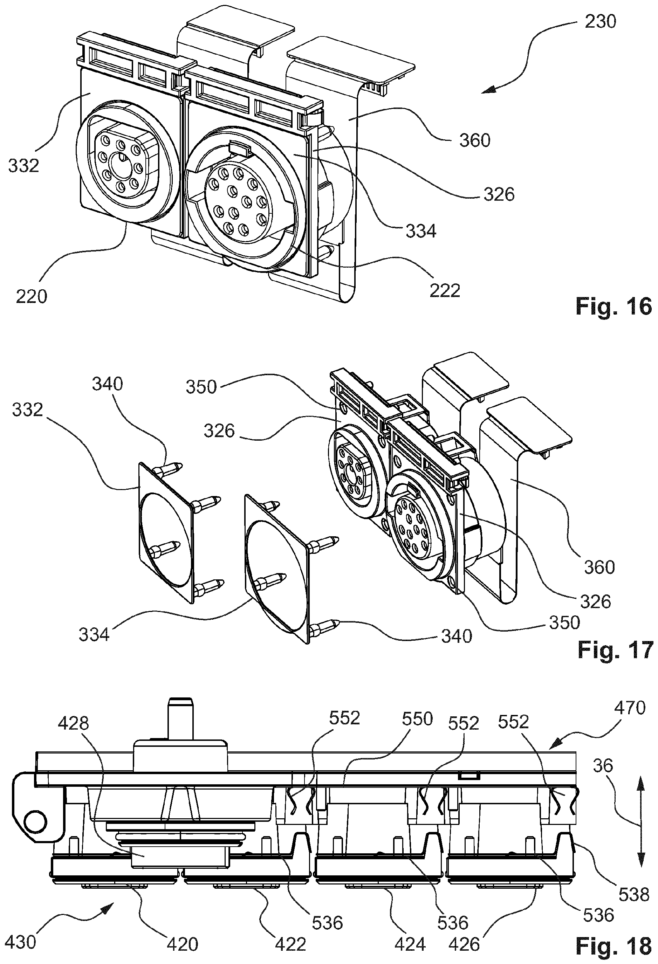

With reference to Figs 16 and 17, an alternative embodiment with regard to the design of the circumferential gasket is elucidated. It is to be noted that the features of the exemplary embodiment of Figs 16 and 17 can be readily combined with the arrangement(s) described in connection with Figs 1 to 15 and in Figs 18 to 20, wherein the circumferential gasket 132 (refer to Fig. 9) would have to be replaced accordingly. For the avoidance of repetitions, primarily differences in the design of the alternative embodiment vis-à-vis the embodiments described herein before will be described in the following. Apart from that, reference is made to Figs 1 to 15 (and to Figs 18 to 20) and the accompanying specification to accomplish the overall design of the connector interface. It is thus clear to the skilled person that respective features can be readily combined to form a connector interface in accordance with the present disclosure.

Fig. 16 and Fig. 17 illustrate perspective views of connector units 220, 222 that form a row 230, whereas the connector units 220, 222 may form part of the overall arrangement of the connector interface 12 of Figs 1 to 15.

In contrast to the arrangement of Fig. 9, the connector units 220, 222 are arranged to be sealed in their abutment position at the connector openings (see reference numerals 56, 58 in Fig. 3) at the case part (see reference numeral 14 in Fig. 3) by a primarily axially sealing circumferential gasket 342, 344. In the exemplary embodiment, gaskets 342, 344 are arranged to be mounted to flanges 326 (of the connector housing) of the connector units 220, 222. In the mounted state, the flanges 326 may urge the gaskets 342, 344 against the case part 14 (Fig. 9 and Fig. 10) into a sealed state.

In certain embodiments, the gaskets 342, 344 comprise snap-in studs 340 as rear protrusions at a relatively flat gasket body, which snap-in studs 340 may be arranged to engage mounting holes 350 at the flange 326 of the connector units 220, 222. The snap-in studs 340 may be provided with locking features (e.g., a widening) that prevents or at least inhibits a pullout of the snap-in studs 340 from the mounting holes 350. Overall, the circumferential gasket 332, 334 may be arranged as molded gaskets having a relatively flat axially effective sealing part and one or more (four in the embodiment as shown in Fig. 17) snap-in studs 340 protruding therefrom to the inner side.

Figs 16 and 17 further show flex foils 360 that are coupled to the connector units 220, 222. Regarding the design of the flex foils 360, there is a great freedom of design when connecting the connector units 220, 222 to circuitry arranged at the inner side.

With reference to Figs 18 to 20, an alternative embodiment with regard to the design of the conductive sheets for dissipating electrostatic charges is elucidated. It is to be noted that the features of the exemplary embodiment of Figs 18 to 20 can be readily combined with the arrangement(s) of the embodiments of Figs 1 to 17. For the avoidance of repetitions, primarily differences in the design of the alternative embodiment vis-à-vis the embodiments described herein before will be described in the following. Apart from that, reference is made to Figs 1 to 17 and the accompanying specification to accomplish the overall design of the connector interface. It is thus clear to the skilled person that respective features can be readily combined to form a connector interface in accordance with the present disclosure.

Figs 18 to 20 each show a partial view of a mounting piece 470 that provides seats for connector units 420, 422, 424, 426, 428, refer in this context to Fig. 8 that shows an insofar similar arrangement of the mounting piece 70 that provides seats 74, 76 for connector units 22, 26, 28. The mounting piece 470 is a mounting aid that secures the connector units 420, 422, 424, 426, 428 to a case part of a connector interface. At least the connector units 420, 422, 424, 426 form a row 430.

Some or all of the connector units 420, 422, 424, 426 are provided with a conductive sheet 536 for dissipating electrostatic charges. For instance, Fig. 14 illustrates an embodiment of a row 30 of connector units having individual conductive sheets 136 that are directly interconnected with neighboring ones for dissipating electrostatic charges. By contrast, in the embodiment as illustrated in Figs 18 to 20, the conductive sheets 536 are mediately conductively interconnected by a connecting bar 550 that is attached to the mounting piece 470. To this end, the conductive sheets 536 may each have a contact projection 538 that engages a receptacle 552 of the connecting bar 550 when the connector units 420 and the mounting piece 470 engage one another in the engagement direction 36.

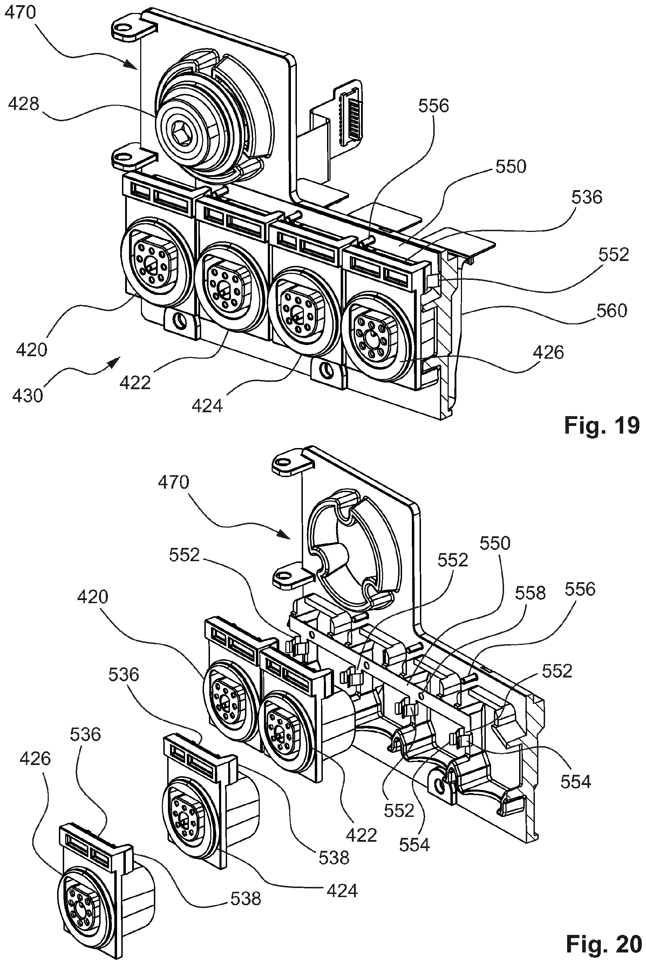

Fig. 19 illustrates an engaged state where the conductive sheets 536 (with their contact projection 538) engage the receptacles 552 of the connecting bar 550. As shown in Fig. 20, the receptacles 552 include two opposite resilient arms 554, whereas the contact projection 538 of the conductive sheets 536 is V-shaped or U-shaped (see the top view of Fig. 18) and pointing at the receptacles 552. Hence, the contact engagement between the conductive sheets 546 and the connecting bar 550 is tolerant to position/shape deviations and tolerant to deformations/vibrations during monitor operation.

As shown in Fig. 20, the connecting bar 550 has one or more mounting recesses 558, that are for instance shaped as mounting holes. At the mounting piece 470, corresponding mounting protrusions 556 are formed that extend towards the outer side. In the mounted state of the connecting bar 550, the mounting protrusions 556 extend through the mounting recesses 558, see also Fig. 19. The mounted state of the connecting bar 550 may be secured by thermally deforming the (typically plastic made) mounting protrusions 556 (e.g., via heat/hot stamping) to secure the connecting bar 550 in a loss-proof manner. In the mounted state, the two resilient arms 554 of a receptacle 552 are slightly urged against the contact projection 538 of the conductive sheet 536. Overall, electrostatic charges can be dissipated via the conductive sheets 536, the connecting bar 550 (and further conductors provided at the device).

Fig. 19 further shows flex foils 560 that are coupled to the connector units 420, 422, 424, 426. Regarding the design of the flex foils 560, there is a great freedom of design when connecting the connector units 420, 422, 424, 426 to circuitry arranged at the inner side.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used as an advantage.

Any reference signs in the claims should not be construed as limiting the scope.

## Claims

1. A connector interface (12) for a patient monitoring module (10), comprising:
- a case part (14) having an outer side (50) and an inner side (52),
- at least one connector opening (56, 58, 60) formed in the case part (14) and extending between the outer side (50) and the inner side (52),
- at least one connector unit (20, 22, 24, 26, 28; 220, 222; 420, 422, 424, 426, 428) that is arranged at the at least one connector opening (56, 58, 60),
wherein the connector unit (20, 22, 24, 26, 28; 420, 422, 424, 426, 428) is accessible through the connector opening (56, 58, 60) from the outer side (50) in an engagement direction (36), and
- a mounting piece (70, 470) having at least one seat (74, 76) for the at least one connector unit (20, 22, 24, 26, 28; 420, 422, 424, 426, 428),
wherein the mounting piece (70, 470) is attached to the case part (14) at the inner side (52) thereof, thereby securing the connector unit (20, 22, 24, 26, 28; 420, 422, 424, 426, 428) in a mounting position with respect to the case part (14).

2. The connector interface (12) as claimed in claim 1, comprising two or more neighboring connector units (20, 22, 24, 26, 28; 420, 422, 424, 426, 428) that are arranged next to one another, wherein two or more connector openings (56, 58, 60) are formed in the case part (14), wherein each of the two or more connector units (20, 22, 24, 26, 28; 420, 422, 424, 426, 428) is arranged at one of the two or more connector openings (56, 58, 60), wherein the mounting piece (70, 470) has two or more seats (74, 76) for the two or more connector units (20, 22, 24, 26, 28; 420, 422, 424, 426, 428), and wherein the mounting piece (70, 470) secures the two or more connector units (20, 22, 24, 26, 28; 420, 422, 424, 426, 428) in a respective mounting position with respect to the case part (14).

3. The connector interface (12) as claimed in claim 2, wherein a divider (40) is arranged on a rear side of the mounting piece (70, 470), the divider (40) forming separate compartments (44) for separate electronics modules (46) that are coupled to the connector units (20, 22, 24, 26, 28; 420, 422, 424, 426, 428), wherein partition walls (42) of the divider (40) are spaced from one another by an offset (48) that is adapted to a width of associated connector units (20, 22, 24, 26, 28; 420, 422, 424, 426, 428).

4. The connector interface (12) as claimed in any one of claims 1-3, wherein the at least one connector unit (20, 22, 24, 26, 28; 420, 422, 424, 426, 428) comprises a connector housing (120), and wherein the mounting piece (70, 470) urges the connector housing (120) against the case part (14).

5. The connector interface (12) as claimed in claim 4, wherein mounting features (112, 114) are formed at the connector housing (120) and the case part (14), which mounting features (112, 114) define a correct mounting orientation for the connector housing (120).

6. The connector interface (12) as claimed in claim 4 or 5, wherein the connector housing (120) comprises a frontal collar (124) and a flange (126), wherein the frontal collar (124) protrudes beyond the flange (126) towards the outer side (50), and wherein preferably at least one of the frontal collar (124) and the flange (126) comprises a positioning aid (84, 86) that is configured to cooperate with a corresponding mating portion (90, 92) formed at the case part (14).

7. The connector interface (12) as claimed in claim 6, wherein the at least one connector unit (20, 22, 24, 26, 28; 420, 422, 424, 426, 428) further comprises a conductive sheet (136, 536) for dissipating electrostatic charges, wherein the flange (126) comprises a support portion (144) to which the conductive sheet (136, 536) is attached.

8. The connector interface (12) as claimed in claim 7, wherein the conductive sheet (136) comprises at least one side contact portion (146) that is configured to contact a side contact portion (146) of a conductive sheet (136) of a neighboring connector unit (20, 22, 24, 26, 28; 420, 422, 424, 426, 428), when two or more connector units (20, 22, 24, 26, 28; 420, 422, 424, 426, 428) are disposed at the case part (14) next to one another.

9. The connector interface (12) as claimed in claim 7, wherein a connecting bar (550) is arranged at the mounting piece (470), wherein the connecting bar (550) comprises two or more resilient receptacles (552), and wherein the conductive sheet (536) contacts one of the two or more resilient receptacles (552) in the mounted state for dissipating electrostatic charges.

10. The connector interface (12) as claimed in any of claims 7-9, wherein at least one mounting protrusion (174) is formed at the support portion (144), and wherein the at least one mounting protrusion (174) engages the conductive sheet (136, 536) and preferably also a flex foil (160) that is contacted by the conductive sheet (136, 536).

11. The connector interface (12) as claimed in any of claims 1-10, wherein the at least one connector unit (20, 22, 24, 26, 28; 420, 422, 424, 426, 428) comprises a contact insert (140), wherein the contact insert (140) has a front contact portion (152) that is arranged to be contacted by a mating connector counterpart (186) through the connector opening (56, 58, 60), wherein the contact insert (140) has a rear contact portion (152), wherein a flex foil (160) is attached to the rear contact portion (152), wherein the flex foil (160) contacts back contacts (162) of the connector unit (20, 22, 24, 26, 28; 420, 422, 424, 426, 428), and wherein the flex foil (160) is arranged to connect the back contacts (162) of the connector unit (20, 22, 24, 26, 28; 420, 422, 424, 426, 428) to an electronics module (46) that is operatively coupled to the connector unit (20, 22, 24, 26, 28; 420, 422, 424, 426, 428).

12. The connector interface (12) as claimed in claims 10 and 11, wherein the flex foil (160) contacts the conductive sheet (136) at the support portion (144) of the flange (126), and wherein preferably a connector housing (120) of the connector unit (20, 22, 24, 26, 28) has a peripheral wall (128) that surrounds the rear contact portion (152) that is encapsulated within the peripheral wall (128).

13. The connector interface (12) as claimed in any of claims 1-12, wherein the connector opening (56, 58, 60) has a centering recess (98, 100, 102) at the inner side (52) and a mouth (104, 106, 108) at the outer side (50), wherein the centering recess (98, 100, 102) is adapted to a centering contour (80, 82) of the connector unit (20, 22, 24, 26, 28; 420, 422, 424, 426, 428), and wherein the mouth (104, 106, 108) forms an access at the outer side (50) having an opening area that is smaller than a cross-sectional area of the centering recess (98, 100, 102).

14. The connector interface (12) as claimed in any of claims 1-13, wherein a circumferential gasket (132; 332, 334) is arranged between the case part (14) and the connector unit (20, 22, 24, 26, 28; 420, 422, 424, 426, 428) that seals an abutment position of the connector unit (20, 22, 24, 26, 28; 420, 422, 424, 426, 428) at the connector opening (56, 58, 60).

15. A patient monitoring module (10) comprising a connector interface (12) as claimed in any of claims 1-14.
